# EUROPEAN PATENT SPECIFICATION

(11) **EP 3 381 395 B1**
(45) Date of publication and mention of the grant of the patent: **10.03.2021**
(21) Application number: 18164194.5
(22) Date of filing: 27.03.2018
(51) Int. Cl.: A61B 18/14, A61B 18/00, A61B 17/00

(54) **CATHETER WITH FLOATING CURVATURE**
KATHETER MIT GLEITENDER KRÜMMUNG
CATHÉTER AVEC COURBURE FLOTTANTE

(30) Priority: 28.03.2017 US 201715471960
(43) Date of publication of application: 03.10.2018
(73) Proprietor: Biosense Webster (Israel) Ltd., Yokneam 2066717 (IL)
(72) Inventor: BAZILIAN, Leonid, Irwindale, CA California 91706 (US)
(74) Representative: Carpmaels & Ransford LLP

(56) References cited:
- EP-A1- 0 842 673
- EP-A2- 0 600 676
- WO-A2-2014/070999

## Description

### FIELD OF INVENTION

This invention relates to electrophysiologic (EP) catheters, in particular, EP catheters for mapping and/or ablation in the heart.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Important sources of undesired signals are located in various tissue regions in or near the heart, for example, the atria and/or and adjacent structures such as areas of the pulmonary veins, and left and right atrial appendages. Regardless of the sources, unwanted signals are conducted abnormally through heart tissue where they can initiate and/or maintain arrhythmia.

Procedures for treating arrhythmia include surgically disrupting the origin of the signals causing the arrhythmia, as well as disrupting the conducting pathways for such signals. More recently, it has been found that by mapping the electrical properties of the heart muscle in conjunction with the heart anatomy, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In a two-step procedure--mapping followed by ablation--electrical activity at points in the heart is typically sensed and measured by advancing a catheter containing one or more electrical sensors into the heart, and acquiring data at a multiplicity of points. These data are then utilized to select the target areas at which ablation is to be performed.

A typical ablation procedure involves the insertion of a catheter having a tip electrode at its distal end into a heart chamber. A reference electrode is provided, generally taped to the patient's skin. Radio frequency (RF) current is applied to the tip electrode, and flows through the surrounding media, i.e., blood and tissue, toward the reference electrode. The distribution of current depends on the amount of electrode surface in contact with the tissue, as compared to blood which has a higher conductivity than the tissue. Heating of the tissue occurs due to its electrical resistivity. If the tissue is heated sufficiently, cellular and other protein destruction ensues; this in turn forms a lesion within the heart muscle which is electrically non-conductive.

A linear or straight catheter works well, for example, when ablating a line of block in the atria. The catheter has a distal tip electrode and perhaps multiple ring electrodes. For effective ablation, the distal tip electrode should have good contact with the target tissue, whether the tissue contact is by a distal face of the tip electrode or a side/circumferential surface of the tip electrode. In either instance, the pressure of the tip electrode against the target tissue may range between about 4 lbs. as a minimum for effective ablation and lesion formation, and about 30 lbs. as a maximum to avoid tissue perforation.

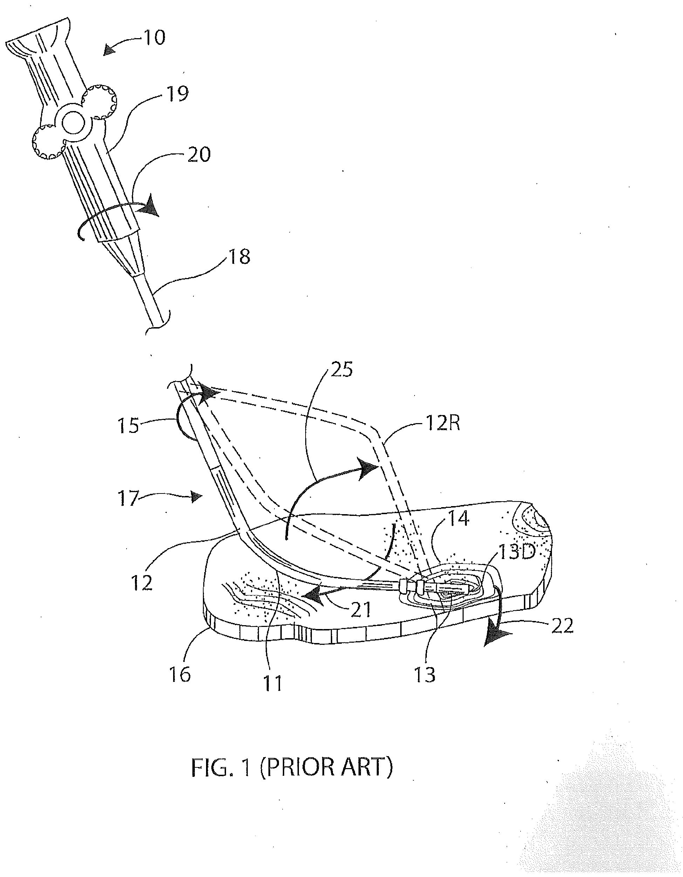

As shown in **FIG. 1****,** a prior art catheter 10 has a distal shaft section 17 advanced inside a patient's body by an operator (not shown) so that it lies against heart tissue 16. To reach target tissue 14, the catheter 10 is manipulated by the operator to provide a deflection curvature 11 in its deflectable section 12 so that a distal tip electrode 13 approaches and contacts the target tissue 14. Typically, where perpendicular orientation is desired, the operator then rotates the distal shaft section 17 (as shown by arrow 15) by rotating a proximal shaft section 18 or a control handle 19 (as shown by arrow 20) in the direction of the deflection for rolling and pivoting the distal tip electrode 13 to slightly stand on its distal face 13D in increasing the pressure of the distal tip electrode 13 against the target tissue 14 for improved tissue contact. However, as the deflectable section 12 is lifted above the tissue surface (to position 12R as shown in broken lines in **FIG. 1**) by rotational force (arrow 25), the distal tip electrode 13 pivoting against the target tissue 14 can become unstable. Often, the rotational force further flip the distal tip electrode and the deflectable section 12 under in the opposite direction (as shown by arrow 21), causing the distal tip electrode to lose contact with the target tissue. Catheter flipping results in loss and disruption of tissue contact and desired contact position, if not also tissue damage from scraping by the distal tip electrode. Moreover, because the flipping occurs when the distal tip is at maximum stiffness and exerting a maximum normal force on the tissue surface, there is an increased risk of tissue perforation. Increasing the stiffness of the distal shaft section 17 may improve stability of the distal tip electrode, but the increased stiffness further increases the risk of tissue perforation.

Accordingly, there is a need for a catheter that can better maintain tissue contact at its distal tip electrode with the application of increased tip contact pressure by an operator, with minimized risk of slippage and tissue perforation.

EP0842673 A1 discloses a stabilized electrophysiology catheter including a main body portion, a flexible and radially deflectable tip portion actuated by longitudinal movements of a manipulator. A plurality of electrodes are positioned along the tip portion. The tip portion includes a main section and a pre-curved, offset, reverse-angle anchor section at its distal end. The anchor section is shaped to engage with and anchor to a coronary opening, typically the coronary sinus opening in the right atrium or a pulmonary vein opening in the left atrium, for stabilized mapping and ablation of the right atrium or the left atrium. A manipulator wire is used to radially deflect the tip portion in a first direction. A core wire is connected at its proximal end to a core wire control element of the catheter, and at its distal end to a tip electrode of the catheter.

### SUMMARY OF THE INVENTION

The present invention providesa catheter as claimed hereinafter. In contrast to prior catheters, whose distal tip or tip electrode can flip, slip and/or lose contact as the orientation of the tip electrode changes due to catheter handle rotation, the catheter of the present invention provide an intermediate section having a floating curvature that enables a tip electrode to remain in tissue contact without significant, if any, migration of the tip electrode on the tissue surface when increased tissue contact pressure is achieved by rotation of the control handle and/or the proximal catheter body.

In some detailed embodiments, the tubing includes a distal plug and a proximal plug in the lumen, and the mandrel extends between the distal plug and the proximal plug.

In some detailed embodiments, the mandrel has shape memory.

In some detailed embodiments, the mandrel includes nitinol.

In some embodiments of the invention, a catheter includes an elongated catheter body, a distal assembly having a tip electrode, and a lumened tubing of greater flexibility between the catheter body and the distal assembly. The catheter also includes a mandrel with an elongated body of a lesser flexibility that is situated in the lumen. Advantageously, the mandrel has a predetermined curvature such that the tubing through which the mandrel extends adopts the predetermined curvature in positioning the distal assembly at an angle from the catheter body. Moreover, the lumen and the mandrel are configured so that the mandrel has freedom of rotational movement within the lumen.

In some detailed embodiments, the mandrel is configured to allow for rotational movement about its respective longitudinal axis that is independent of rotational movement of the tubing.

In some detailed embodiments, the mandrel is configured to allow for rotational movement about its respective longitudinal axis that is independent of rotation of the tubing about its respective longitudinal axis.

In some detailed embodiments, the mandrel is configured to allow for rotation about its respective longitudinal axis that is independent of rotation of the tubing about its respective longitudinal axis.

In some detailed embodiments, the mandrel is configured to allow for rotational movement about its respective longitudinal axis without rotation of the tubing about its respective longitudinal axis.

### BRIEF DESCRIPTION OF THE DRAWINGS

These and other features and advantages of the present invention will be better understood by reference to the following detailed description when considered in conjunction with the accompanying drawings wherein:
FIG. 1 is an illustration of a prior art catheter having a deflected section and a distal tip electrode in contact with target tissue.
FIG. 2 is an illustration of a catheter in accordance with some embodiments of the present invention, in contact with tissue.
FIG. 3 is an end cross-sectional view of the catheter of FIG. 2, taken along line A-A.
FIG. 4 is an end cross-sectional view of the catheter of FIG. 2, taken along line B-B.
FIG. 5A is a pre-curved section with a lumened tubing and a mandrel, in accordance with some embodiments of the present invention.
FIG. 5B is a pre-curved section with a lumened tubing and a mandrel, in accordance with other embodiments of the present invention.
FIG. 6 is an illustration of a catheter in accordance with an embodiment of the present invention, exhibiting distinctive behaviors and/or characteristics.
FIG. 7 is a pre-curved section with a lumened tubing and multiple mandrels, in accordance with other additional embodiments of the present invention.
FIG. 8 is a pre-curved section with a lumened tubing and multiple mandrels, in accordance with further additional embodiments of the present invention.
FIG. 9 is an end cross-sectional view of a distal assembly of a catheter, in accordance with some embodiments of the present invention.

### DETAILED DESCRIPTION OF THE INVENTION

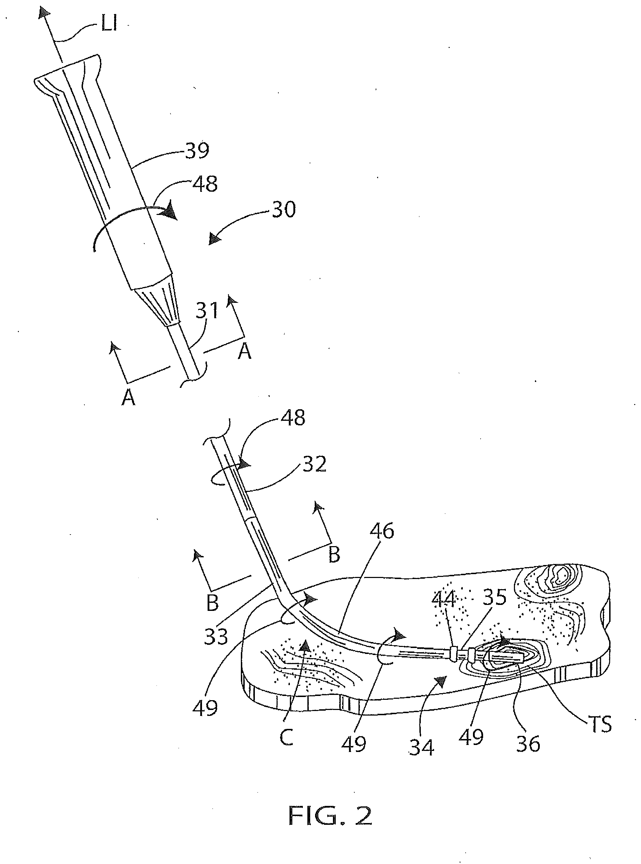

In some embodiments of the present invention as shown in **FIG. 2****,** the catheter 30 has an elongated catheter shaft 31 with a proximal catheter body 32, a distal assembly 34, and an intermediate "pre-curved" section 33 with a predetermined and preformed curvature C extending between the catheter body 32 and the distal assembly 34 which positions the distal assembly 34 at an angle from the proximal catheter body 32. The distal assembly 34 includes a distal tip electrode 36 adapted for tissue contact. The distal assembly 34 may also include one or more ring electrodes 44 carried on a connector section 35 extending between the section 33 and the distal tip electrode 36. The catheter 30 also includes a control handle 39 attached to the proximal end of the catheter body 32.

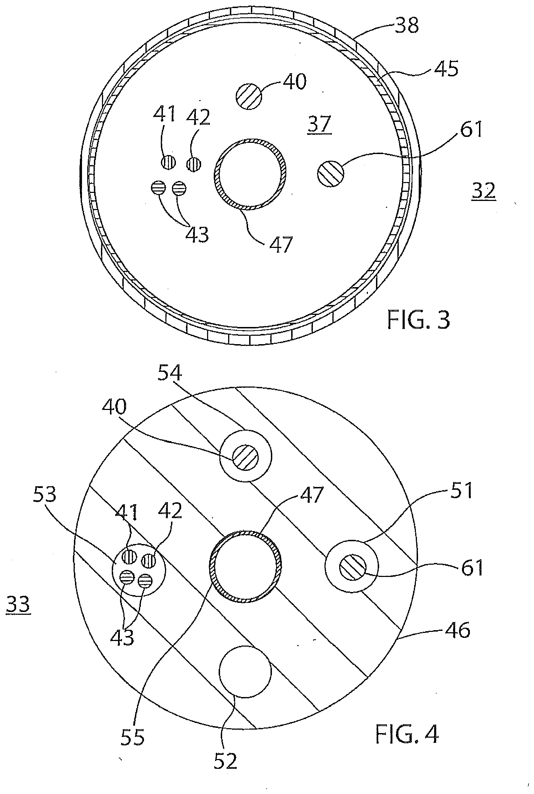

In some embodiments, the catheter body 32 has an elongated tubular construction having a single, axial or central lumen 37, as shown in **FIG. 3****.** The catheter body 32 is flexible, i.e., bendable, but substantially non-compressible along its length. The catheter body 32 can be of any suitable construction and made of any suitable material. In some embodiments, the catheter body 32 comprises an outer wall 38 made of polyurethane or PEBAX. The outer wall 38 comprises an imbedded braided mesh of stainless steel or the like to increase torsional stiffness of the catheter body 32 so that, when the control handle 39 is rotated, as shown in **FIG. 2****,** entire length of the shaft 31 rotates in a corresponding manner. The inner surface of the outer wall 38 may be lined with a stiffening tube 45 to provide improved torsional stability.

The outer diameter of the catheter body 32 is not critical. Likewise, the thickness of the outer wall 38 is not critical, but is thin enough so that the central lumen 37 can accommodate a variety of components, including for example, a position sensor cable assembly 40, including one or more single axis sensors (SAS) carried in or near the distal assembly 34, as shown in **FIG. 3****.** The components may also include wire pair 41 and 42 of different metals for the distal tip electrode 36, lead wires 43 for the ring electrodes 44, and an irrigation tubing 47 for passing irrigation fluid along the catheter shaft to the distal tip electrode 36. Ablation energy, e.g., RF energy, is delivered to the distal tip electrode 36 via the wire 41, for example, a copper wire, number "40," whereas the wire 41 and the wire 42, for example, a constantan wire, together function as a thermocouple sensing the temperature of the distal tip electrode 36. The lead wires 43 may transmit electrical signals sensed by the ring electrodes 44 toward the control handle for processing.

The pre-curved section 33 comprises a shorter section of tubing 46 having at least one lumen. In some embodiments, the tubing 46 has multiple lumens, including, off-axis lumens 51, 52, 53, 54, as shown in **FIG. 4****.** The tubing 46 may also include center lumen 55. In some embodiments, the irrigation tubing 47 passes through center lumen 55, the wires 41, 42, and 43 pass through the lumen 53, and the position cable assembly 40 passes through the lumen 57 with the SAS (not shown) positioned at or near the distal end of the tubing 46.

Passing through the lumen 51 is a mandrel 61 having an elongated body preformed with a predetermined curvature C. The elongated body has a greater stiffness/lesser flexibility relative to the tubing 46. In some embodiments, the mandrel is constructed of a flexible wire, for example, stainless steel wire, such as "P305SS," having a greater durometer or stiffness than the multi-lumened tubing 46. In that regard, the mandrel 61 when inserted into the lumen 51 imparts the predetermined curvature to the tubing 46, resulting in the tubing 46 generally adopting the predetermined curvature such that a longitudinal axis of the catheter body 32 and a longitudinal axis of the distal assembly 34 do not lie on a single line but are angularly offset and unaligned with each other.

As shown in **FIG. 2****,** the pre-curved section 33 with the predetermined curvature C facilitates the distal assembly 34 to be maneuvered by an operator to reach target site TS of patient tissue. More importantly, the predetermined curvature of the pre-curved section 33, as provided by the mandrel 61, allows the operator to increase contact pressure of the distal tip electrode 36 against the tissue of the target site TS without significantly changing the angle of the distal tip electrode 36 relative to the tissue. In contrast to prior catheters with one or more anchored deflection puller wires, the catheter 10, as a feature of the present invention, can generally maintain the angle of the distal tip electrode 36 and the pre-curved section 33 to the tissue by advantageously rotating along its entire length about its longitudinal axis, even through the length of the pre-curved section 33, when the operator increases contact pressure by rotating the control handle 39 and/or the proximal catheter body 31. That is, instead of the distal tip electrode 36 pivoting and the pre-curved section 33 lifting above tissue surface and then instantaneously flipping over (as shown in **FIG. 1**), the pre-curved section 33 and the distal tip electrode 36 maintain their angle and thus stability with the tissue by rotating about their longitudinal axis. To allow this rotation, the mandrel 61 along its entire body is advantageously unfixed and detached from the catheter and thus able to exhibit a "floating" behavior within the lumen 51, unlike the puller wires of conventional deflectable catheters of **FIG. 1** which are anchored at their distal and proximal ends to the control handle 19 and the distal shaft section 17, respectively.

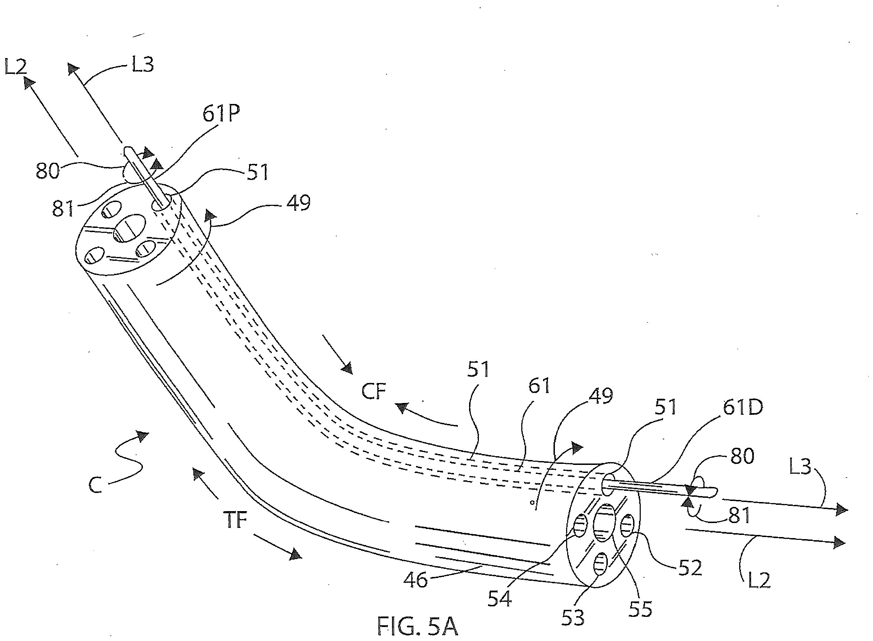

In some embodiments, the lumen 51 is the lumen closest to the inner circumference of the predetermined curvature C as shown in **FIG. 5A****.** With the mandrel 61 unfixed and detached to the tubing 46 and the lumen 51 (and thus "floating in the lumen"), the mandrel imparts the predetermined curvature to the tubing 46 while being prevented from storing and/or imparting to the tubing 46 any significant rotational force that can cause undesirable behavior in the catheter, as explained below in further detail.

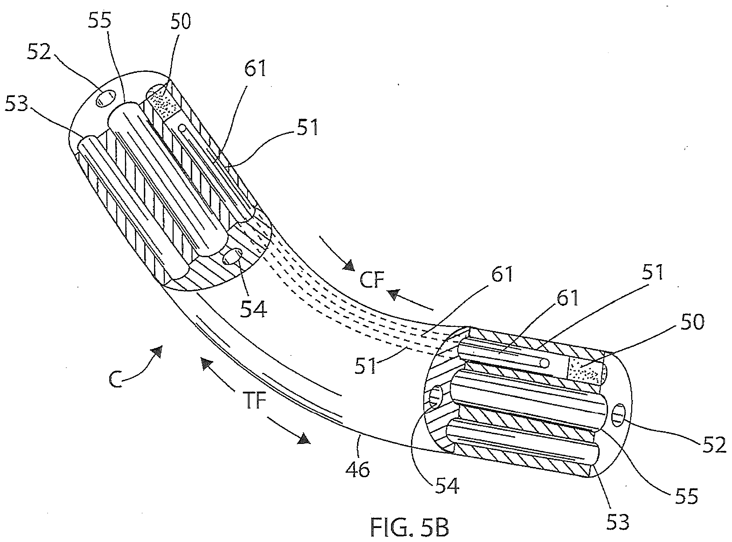

In some embodiments, the length of the mandrel 61 is generally limited to a length no greater than the length of the catheter shaft 31, and preferably to a length no lesser than the length of the pre-curved section 33. In the embodiment of **FIG. 5A****,** the mandrel 61 has a length slightly greater than the tubing 46 such that distal end 61D and proximal 61P extend slightly beyond the distal end and proximal end of the tubing 46. In some embodiments as shown in **FIG. 5B****,** the length of the mandrel 61 is slightly less than the length of the multi-lumened tubing 46 so that a distal end and the proximal end of the lumen 51 may each include a plug 50 to contain the mandrel 61 within the lumen 51 longitudinally. The plugs do not interfere with the ability of the mandrel to "float" in the lumen 51. Without excessive length extending into the central lumen 37 of the catheter body 32, the mandrel 61 is contained within the lumen 51 and thus avoids entanglement with other components in the central lumen 37 in the catheter body 32 (for example, the irrigation tubing 47, the wires 41, 42 and 43, and thus more freely allows the rotational force traveling along the catheter from the control handle 39 to continue distally to the multi-lumen tubing 46 and the distal tip electrode 37.

When a sufficient rotational force reaches the tubing 46 and the mandrel 61, the tubing 46 being sufficiently more flexible and pliable than the mandrel 61 experiences a dynamic compression force DC along the inner circumference of the predetermined curvature and a dynamic tension force DT along the outer circumference of the predetermined curvature in rolling the tubing 46 about longitudinal axis L2, as shown by arrow 49 ("rolling" and "rotating" used interchangeably herein). In that regard, the relative cross-section and/or diameter of the lumen 51 and the mandrel 61, and/or low-friction surfaces between the lumen and the mandrel (including, for example, a lubricious coating, such as TEFLON, may be applied to the mandrel) allow the tubing 46 to simultaneously roll while conforming to the curvature imparted by the mandrel. These features allow movements, including rotational movement, of the tubing 46 and the mandrel 61 to be decoupled and generally independent of each other, so that the mandrel can "float" and the rotational force can reach the distal tip electrode 36 to increase contact pressure. As such, no significant rotational energy can be stored in the mandrel to be released as torque dislodging and flipping the tubing 46 and distal tip electrode 36. In that regard, the tubing 46 is constructed of any suitable material, with sufficient elastic flexibility, including, e.g., PELLETHANE, that is softer or more pliable relative to the mandrel 61 and can adopt a curved configuration imparted by the mandrel 61.

In contrast, it is understood that a catheter with a longer mandrel that extends into the catheter body 32 and/or a mandrel anchored or fixed to the catheter at any one or more points on its body or length poses a significantly greater resistance, if not obstruction, to ability of the rotational force to travel along the catheter from the control handle 39 to the pre-curved section 33 and the distal tip electrode 36. Such resistance and obstruction is most likely to create a sufficient torque that lifts the pre-curved section 33 and destabilizes the distal tip, causing the distal tip electrode to slip and the curved section to flip.

The mechanism of the mandrel and the tubing 46 is described in further detail with reference to **FIG. 2****.** With the distal tip electrode 36, for example, resting on tissue surface, the predetermined curvature of the pre-curved section 33 is statically imparted by the mandrel 61 so that the tubing 46 experiences static compression and tension in adopting the curvature of the mandrel 61. As the operator advances the catheter distally for the purpose of increasing the normal (contact) force of the distal tip on the tissue surface (such as in the pursuit of improved lesion formation by RF ablation by the distal tip electrode 36), the static compression and tension in the tubing 46 may be further enhanced and enforced as applied by the operator. As the operator rotates the control handle 39 and/or the catheter body 32 about its longitudinal axis L1 in the direction of toward the inner circumference (arrow 48 in **FIG. 2**), the rotational force reaches the tubing 46, and the tubing 46 experiences dynamic compression and tension as it reacts with rolling (or rotational movement) in the same direction (arrows 49) about its longitudinal axis L2 while remaining in conformity with the curvature of the mandrel 61. Inside the lumen 51 of the rotating tubing 46 which causes the lumen 51 to "orbit" the longitudinal axis L2, the mandrel 61 may begin to rotate about its own longitudinal axis L3. However, because the mandrel 61 is significantly stiffer than the tubing 46, such rotation of the mandrel within the lumen about its own longitudinal axis L3 is limited by a number of features, including (1) its own predetermined curvature becoming misaligned with the enforced/enhanced curvature of the tubing 46 as the lumen 51 "orbits" the longitudinal axis L2, (2) the lubricious surface(s) of the mandrel 61 and/or the lumen 51 in prevent the tubing 46 from gripping the mandrel 61 (and vice versa), and/or (3) appropriate sizing and configuration of the cross-section and/or diameter of the lumen 51 and the mandrel 61. These features when appropriately balanced with each other allow the mandrel 61 to "float" in the lumen 51 of the rolling tubing 46 and maintain the predetermined curvature of the pre-curved section in stabilizing the distal tip electrode in contact with tissue.

Thus, with any rotation of the mandrel 61 about its own longitudinal axis L3 in the same direction as the rotation of the tubing 46 (see arrow 81), the mandrel 61 being advantageously detached, unfixed and unanchored to the tubing 46 or any other part of the catheter can readjust as often as needed by "popping" in momentary accelerated rotational movement either backwards in the opposite direction (arrow 80) or forward (arrow 81) in the same direction as the rotation of tubing (arrow 49) so that the mandrel can return to or remain in alignment with the enforced curvature of the lumen 51 and the tubing 46 as imposed by the dynamic tension and compression forces imparted by the operator while he is holding the catheter distal tip electrode 36 against the tissue surface and rotating the catheter along its longitudinal axis L1. It is understood that "rotation" may encompass or results from a series or plurality of "rotational movements," although these terms are used interchangeably herein as appropriate and relevant.

The mandrel 61 can momentarily adjust itself within the lumen 51 to realign with the curvature of the tubing 46 in a number of ways, for example, by resisting rotational movement or rotation followed by either momentarily reversing rotation (arrow 80) into alignment or momentarily accelerating forward rotation (arrow 81) into alignment, in a manner that advantageously avoids disruption of the distal tip electrode 36 continuous contact with tissue with minimal migration of the distal tip electrode 36 away from the target site TS, as shown in **FIG. 2****.** Such rotational movement or rotation in adjustment between the mandrel 61 and the tubing 46 includes, for example, different direction of rotation and different rotational speed. However, it is such freedom of rotational movement or rotation in the mandrel 61 relative to the tubing 46 that enables the rotational movement or rotation of the distal tip electrode 46 to be responsive to the rotational movement or rotation of the section 33 and the proximal catheter body 32.

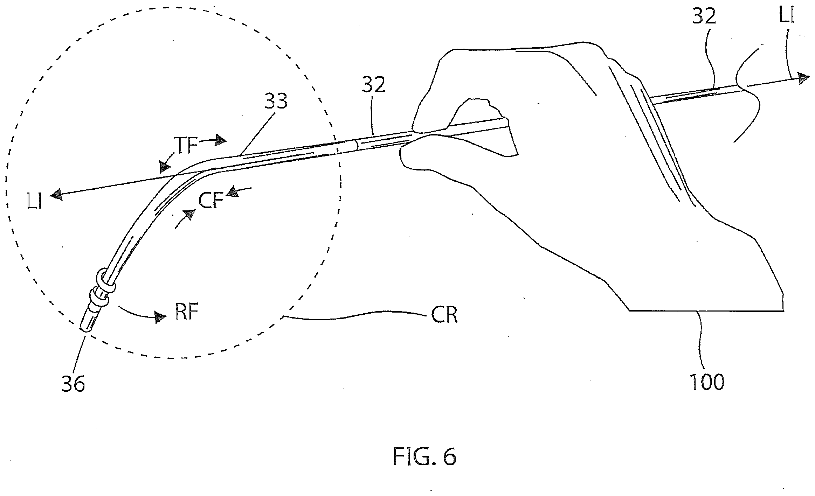

In some embodiments of the present invention, a catheter having a "floating" curvature provided by a detached or unfixed mandrel with a lesser flexibility and a predetermined curvature imparted to a tubing of greater flexibility also exhibits distinctive characteristics which can be described in reference to **FIG. 6****.** With the proximal catheter body 32 of the catheter fixed against rotation about its longitudinal axis L1 (such as when gripped by a hand 100 of an operator), the predetermined curvature of the pre-curved section 33 is nevertheless movably/rotatably responsive to a rotational force RF lying in the plane perpendicular to the axis L1. The curvature of the pre-curved section 33 which is shown to face outwardly from the axis L1 is able to "float" or continue facing outwardly, despite the rotation of the pre-curved section 33 about the longitudinal axis L1, in a manner where the distal tip electrode 36 traces a circle CR that lies in the plane generally perpendicular to the longitudinal axis L1 and whose center is intersected by the axis L1. The pre-curved section 33 is able to resemble in appearance the spinning motion of an airplane propeller despite the inability of the proximal end of the tubing 46 of the pre-curved section 33 to actually spin or rotate because it is fixed to the distal end of the tubing of the catheter body 32 which is gripped against any rotation by the hand 100 of the operator. Notably, the pre-curved section 33 does not unwind or spin backwards upon removal of the rotational force RF because it does not store any rotational energy. This "floating" action is due to aforementioned features factors which allow the sidewalls of the tubing 46 to react to dynamic compression and tension forces so that the tubing 46 can comply with the predetermined curvature of the mandrel. In a similar manner as described above, the lumen 51 and/or the mandrel 61 are configured so that the mandrel 61 can "float" in the lumen 51 regardless of the actions of the tubing 46 and, if needed, adjust itself within the lumen 51 with momentary accelerated rotational movements in a backward direction from the spinning direction and/or momentary accelerated movements in the same direction as the spinning direction. In contrast, a conventional catheter in a deflected curvature enabled by a puller wire anchored at both its distal and proximal ends to the catheter is unable to spin (or appear to spin) without significantly changing the deflected curvature and/or breaking the puller wire. Alternatively, where a conventional catheter allows "propeller spinning," the conventional catheter would also be storing rotational energy such that the catheter would immediately unwind upon removal of the rotational force RF.

It is understood that the cross-section and/or diameter of the lumen 51 are sized relative to the cross-section and/or diameter of the mandrel 61 to provide sufficient room for the mandrel 61 the freedom to move and adjust as needed. No matter how many rotations the tubing 46 may undergo, the mandrel 61 continues to adjust as needed thus providing the pre-curved section 33 with a "floating curvature" that enables the distal tip electrode 36 to remain in contact with the tissue surface without significant migration from the target tissue site. The "floating" curvature is able to continue so long as any portions of the mandrel 61 extending beyond the lumen 51 (distally and/or proximally) remain untangled with other components in the catheter and the mandrel otherwise avoids storing rotational energy that can dislodge the distal tip electrode 46 from contact with the tissue surface.

It is understood that for the catheter to exhibit optimum "floating curvature" there is a balance between the flexibility of the tubing 46 and the flexibility of the mandrel 61, so that the tubing 46 can deform in compression and tension so as to allow the tubing 46 to roll, as shown in **FIG. 5A**) while the mandrel has sufficiently flexibility to flex and bend as needed in order to adjust itself within an appropriately sized lumen 51. Flexibility of the tubing and the mandrel depends on a number of factors, including their construction material, and their relative cross-section and/or diameter. In that regard, a suitable material for constructing the mandrel is an elastically flexible material or a material with shape-memory, including, e.g., nitinol. In the absence of an external force, a preformed body with elastic flexibility or shape-memory maintains its original configuration. When subjected to an external force, the body elastically flexes or deforms into a different configuration in response to the external force. When the external force is abated or removed, the body returns to its original configuration.

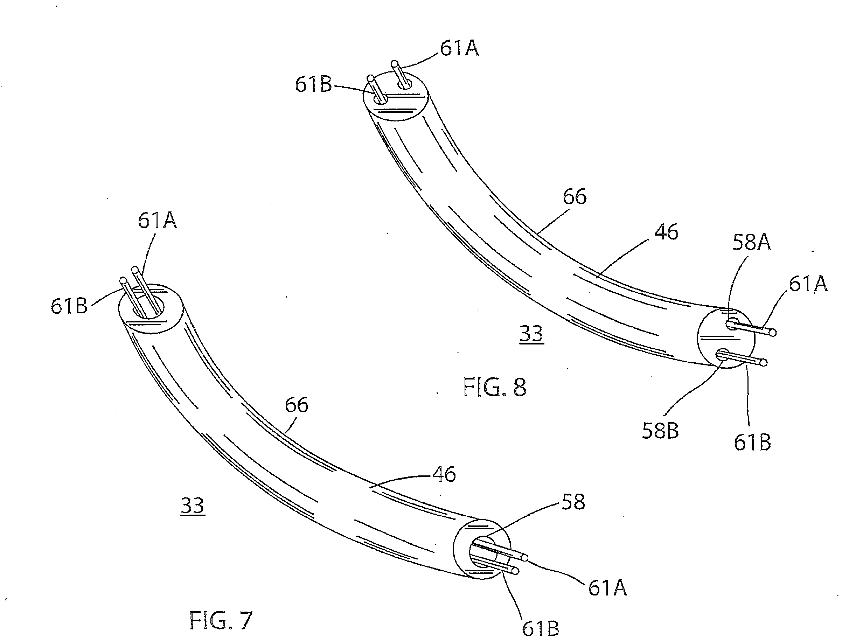

It is understood that in other embodiments of the present invention, the pre-curved section 33 may include more than one mandrel as desired or appropriate. In embodiments having at least two generally similar mandrels, mandrels 61A and 61B may both occupy a lumen 58 of the tubing 46, as shown in **FIG. 7****,** provided there is sufficient room with in the lumen 58 to allow each mandrel to float without significantly entangling or interfering with each other. In other embodiments, as shown in **FIG. 8****,** each mandrel 61A and 62B occupies a respective lumen 58A and 58B. In each of these embodiments, the mandrels are oriented in a similar fashion such that the mandrels impart their curvature to the tubing 46 in a symbiotic manner. In some of these embodiments, the lumen closest to the inner circumference 66 of the pre-curved section 33 is occupied by at least one mandrel for optimum performance.

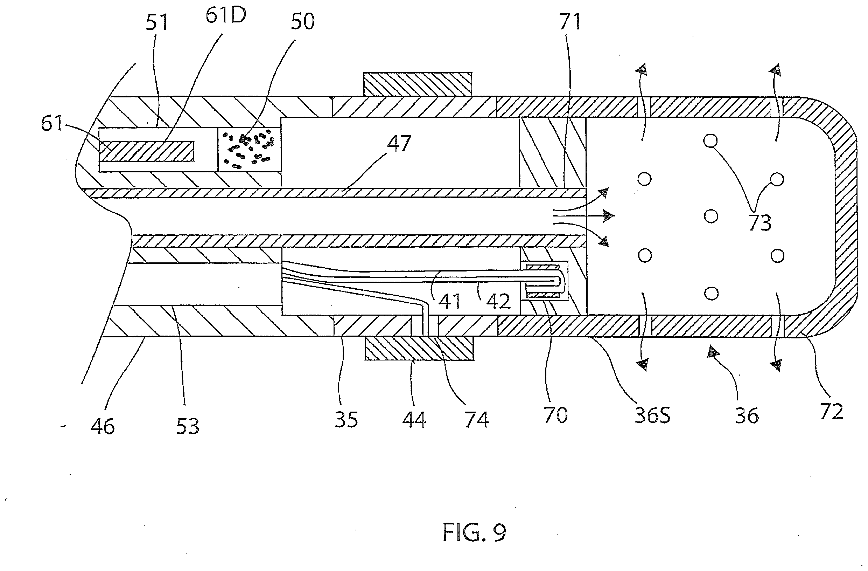

In some embodiments, the connector section 35 is a short tubing extending between the curved section 33 and the distal tip electrode 36, as shown in **FIG. 9****.** The lumen of the connector section 35 allows components extending from the lumens of the pre-curved section 33 to reorient themselves as needed to reach connected components in the distal assembly 34. For example, the one or more lead wires 43 reach one or more respective ring electrodes 44 carried on the outer surface of the connector section 35 via through-holes 74 formed in the side wall of the connector section 35, the wire pair 41/42 reach a blind hole 70 formed in the tip electrode 36, the irrigation tubing reach a through-hole 71 formed in the tip electrode 36 whose thin shell 72 is formed with a plurality of irrigation apertures 73 for passing fluid delivered by the irrigation tubing to outside the tip electrode 36.

In use, a suitable guiding sheath (not shown) is inserted into the patient with its distal end positioned at or near a desired tissue location for diagnostics such as mapping and/or treatment such as ablation. An example of a suitable guiding sheath for use in connection with the present invention is the Preface Braided Guiding Sheath, commercially available from Biosense Webster, Inc. (Diamond Bar, Calif.). The pre-curved section 33 of the catheter 30 is temporarily straightened by an operator who feeds the distal tip electrode 36 into the guiding sheath, followed by the temporarily-straightened pre-curved section 33, and further followed by the catheter body 32. When the distal tip electrode 36 and the pre-curved section 33 are advanced past a distal end of the guiding sheath, the pre-curved section 33 returns to its curved configuration and the operator manipulates the catheter until the distal tip electrode 36 lies against tissue with the distal tip electrode 36 positioned in contact with the target tissue. Advantageously, the operator may apply a contact pressure ranging between about 4-30 lbs.

The preceding description has been presented with reference to presently disclosed embodiments of the invention. Workers skilled in the art and technology to which this invention pertains will appreciate that alterations and changes in the described structure may be practiced without meaningfully departing from the scope of this invention as defined by the claims. As understood by one of ordinary skill in the art, the drawings are not necessarily to scale, and any one or more features or combinations of features described in any one or more embodiments may be incorporated into any other one or more embodiments or combined with any one or more feature(s) of other embodiments, as desired or needed. Accordingly, the foregoing description should not be read as pertaining only to the precise structures described and illustrated in the accompanying drawings, but rather should be read consistent with and as support to the following claims.

## Claims

1. A catheter (30) comprising:
an elongated catheter body (31);
a distal assembly (34) having a tip electrode (36);
a tubing (46) between the catheter body and the distal assembly, the tubing having a lumen (51), the tubing having a first flexibility;
a mandrel (61) having an elongated body with a second flexibility less than the first flexibility, the elongated body situated in the lumen and having a predetermined curvature,
wherein the tubing is configured to adopt the predetermined curvature and allow the mandrel rotational movement within the lumer, **characterised in** the mandrel being unfixed to and detached from the catheter along the entire elongated body of said mandrel.

2. The catheter of claim 1, wherein the mandrel is configured to allow for rotational movement about its respective longitudinal axis that is independent of rotational movement of the tubing.

3. The catheter of claim 1, wherein the mandrel is configured to allow for rotational movement about its respective longitudinal axis that is independent of rotation of the tubing about its respective longitudinal axis.

4. The catheter of claim 1, wherein the mandrel is configured to allow for rotation about its respective longitudinal axis that is independent of rotation of the tubing about its respective longitudinal axis.

5. The catheter of claim 1, wherein the mandrel is configured to allow for rotational movement about its respective longitudinal axis without rotation of the tubing about its respective longitudinal axis.

6. The catheter of claim 1, wherein the mandrel is configured to allow for a rotational direction about its respective longitudinal axis within the lumen that is independent of a rotational direction of the tubing about its respective longitudinal axis.

7. The catheter of claim 1, wherein the mandrel is configured to allow for a rotational speed about its respective longitudinal axis within the lumen that is independent of a rotational speed of the tubing about its respective longitudinal axis.

8. The catheter of claim 1,
wherein the tip electrode is configured for rotation about a first longitudinal axis in response to rotation of the catheter body about a second longitudinal axis different from the first longitudinal axis.

9. The catheter of claim 8, wherein the rotation of the tip electrode is in the same direction as the rotation of the catheter body.

10. The catheter of claim 8, wherein the rotation of the tip electrode is at the same speed as the rotation of the catheter body.

11. The catheter of claim 8, wherein the tip electrode is configured for tissue contact without migration of the tip electrode relative to the tissue.

12. The catheter of claim 1 or claim 8, wherein the tubing includes a distal plug (50) and a proximal plug (50) in the lumen, and the mandrel extends between the distal plug and the proximal plug.

13. The catheter of claim 12, wherein the mandrel is configured to allow rotational movement between the distal and proximal plugs.

14. The catheter of claim 1 or claim 8, wherein the mandrel has shape memory.

15. The catheter of claim 1 or claim 8, wherein the mandrel includes nitinol.

## Patentansprüche

1. Katheter (30), umfassend:
einen länglichen Katheterkörper (31);
eine distale Anordnung (34) mit einer Spitzenelektrode (36);
einen Schlauch (46) zwischen dem Katheterkörper und der distalen Anordnung, wobei der Schlauch ein Lumen (51) aufweist, wobei der Schlauch eine erste Flexibilität aufweist;
einen Dorn (61), der einen länglichen Körper mit einer zweiten Flexibilität aufweist, die kleiner ist als die erste Flexibilität, wobei sich der längliche Körper in dem Lumen befindet und eine vorbestimmte Krümmung aufweist,
wobei der Schlauch dazu ausgelegt ist, die vorbestimmte Krümmung anzunehmen und eine Drehbewegung des Dorns im Innern des Lumens zu ermöglichen, **dadurch gekennzeichnet, dass** der Dorn entlang des gesamten länglichen Körpers des Dorns nicht an dem Katheter befestigt, sondern davon gelöst ist.

2. Katheter nach Anspruch 1, wobei der Dorn dazu ausgelegt ist, eine Drehbewegung um seine jeweilige Längsachse zu ermöglichen, die unabhängig von der Drehbewegung des Schlauchs ist.

3. Katheter nach Anspruch 1, wobei der Dorn dazu ausgelegt ist, eine Drehbewegung um seine jeweilige Längsachse zu ermöglichen, die unabhängig von der Drehbewegung des Schlauchs um seine jeweilige Längsachse ist.

4. Katheter nach Anspruch 1, wobei der Dorn dazu ausgelegt ist, eine Drehbewegung um seine jeweilige Längsachse zu ermöglichen, die unabhängig von der Drehbewegung des Schlauchs um seine jeweilige Längsachse ist.

5. Katheter nach Anspruch 1, wobei der Dorn dazu ausgelegt ist, eine Drehbewegung um seine jeweilige Längsachse zu ermöglichen, die unabhängig von der Drehbewegung des Schlauchs um seine jeweilige Längsachse ist.

6. Katheter nach Anspruch 1, wobei der Dorn dazu ausgelegt ist, eine Drehrichtung um seine jeweilige Längsachse im Innern des Lumens zu ermöglichen, die von einer Drehrichtung des Schlauchs um seine jeweilige Längsachse unabhängig ist.

7. Katheter nach Anspruch 1, wobei der Dorn dazu ausgelegt ist, eine Drehrichtung um seine jeweilige Längsachse im Innern des Lumens zu ermöglichen, die von einer Drehrichtung des Schlauchs um seine jeweilige Längsachse unabhängig ist.

8. Katheter nach Anspruch 1,
wobei die Spitzenelektrode zur Drehung um eine erste Längsachse in Reaktion auf die Drehung des Katheterkörpers um eine zweite Längsachse ausgelegt ist, die sich von der ersten Längsachse unterscheidet.

9. Katheter nach Anspruch 8, wobei die Drehung der Spitzenelektrode in dieselbe Richtung erfolgt wie die Drehung des Katheterkörpers.

10. Katheter nach Anspruch 8, wobei die Drehung der Spitzenelektrode in derselben Geschwindigkeit erfolgt wie die Drehung des Katheterkörpers.

11. Katheter nach Anspruch 8, wobei die Spitzenelektrode für Gewebekontakt ohne Migration der Spitzenelektrode bezogen auf das Gewebe ausgelegt ist.

12. Katheter nach Anspruch 1 oder Anspruch 8, wobei der Schlauch einen distalen Stopfen (50) und einen proximalen Stopfen (50) in dem Lumen umfasst und sich der Dorn zwischen dem distalen Stopfen und dem proximalen Stopfen erstreckt.

13. Katheter nach Anspruch 12, wobei der Dorn dazu ausgelegt ist, eine Drehbewegung zwischen dem distalen und dem proximalen Stopfen zu ermöglichen.

14. Katheter nach Anspruch 1 oder Anspruch 8, wobei der Dorn ein Formgedächtnis aufweist.

15. Katheter nach Anspruch 1 oder Anspruch 8, wobei der Dorn Nitinol umfasst.

## Revendications

1. Cathéter (30) comprenant :
un corps de cathéter allongé (31) ;
un ensemble distal (34) comportant une électrode de pointe (36) ;
un tube (46) entre le corps de cathéter et l'ensemble distal, le tube possédant une lumière (51), le tube ayant une première flexibilité ;
un mandrin (61) comportant un corps allongé ayant une seconde flexibilité inférieure à la première flexibilité, le corps allongé étant situé dans la lumière et ayant une courbure prédéfinie,
dans lequel le tube est configuré pour adopter la courbure prédéfinie et permettre le mouvement de rotation du mandrin à l'intérieur de la lumière, **caractérisé par** le mandrin étant non fixé au cathéter et détaché de celui-ci sur tout le corps allongé dudit mandrin.

2. Cathéter selon la revendication 1, dans lequel le mandrin est configuré pour permettre un mouvement de rotation autour de son axe longitudinal respectif qui est indépendant d'un mouvement de rotation du tube.

3. Cathéter selon la revendication 1, dans lequel le mandrin est configuré pour permettre un mouvement de rotation autour de son axe longitudinal respectif qui est indépendant d'une rotation du tube autour de son axe longitudinal respectif.

4. Cathéter selon la revendication 1, dans lequel le mandrin est configuré pour permettre une rotation autour de son axe longitudinal respectif qui est indépendante d'une rotation du tube autour de son axe longitudinal respectif.

5. Cathéter selon la revendication 1, dans lequel le mandrin est configuré pour permettre un mouvement de rotation autour de son axe longitudinal respectif sans rotation du tube autour de son axe longitudinal respectif.

6. Cathéter selon la revendication 1, dans lequel le mandrin est configuré pour permettre une direction de rotation autour de son axe longitudinal respectif à l'intérieur de la lumière qui est indépendante d'une direction de rotation du tube autour de son axe longitudinal respectif.

7. Cathéter selon la revendication 1, dans lequel le mandrin est configuré pour permettre une vitesse de rotation autour de son axe longitudinal respectif à l'intérieur de la lumière qui est indépendante d'une vitesse de rotation du tube autour de son axe longitudinal respectif.

8. Cathéter selon la revendication 1,
dans lequel l'électrode de pointe est conçue pour une rotation autour de son premier axe longitudinal en réponse à la rotation du corps de cathéter autour d'un second axe longitudinal différent du premier axe longitudinal.

9. Cathéter selon la revendication 8, dans lequel la rotation de l'électrode de pointe est dans la même direction que la rotation du corps de cathéter.

10. Cathéter selon la revendication 8, dans lequel la rotation de l'électrode de pointe est à la même vitesse que la rotation du corps de cathéter.

11. Cathéter selon la revendication 8, dans lequel l'électrode de pointe est conçue pour un contact avec un tissu sans migration de l'électrode de pointe par rapport au tissu.

12. Cathéter selon la revendication 1 ou la revendication 8, dans lequel le tube comprend un bouchon distal (50) et un bouchon proximal (50) dans la lumière, et le mandrin s'étend entre le bouchon distal et le bouchon proximal.

13. Cathéter selon la revendication 12, dans lequel le mandrin est configuré pour permettre un mouvement de rotation entre les bouchons distal et proximal.

14. Cathéter selon la revendication 1 ou la revendication 8, dans lequel le mandrin a une mémoire de forme.

15. Cathéter selon la revendication 1 ou la revendication 8, dans lequel le mandrin comprend du nitinol.
